# FASCICULE DE BREVET EUROPEEN

(11) **EP 0 489 140 B1**
(45) Date de publication et mention de la délivrance du brevet: **02.08.1995**
(21) Numéro de dépôt: 91912175.6
(22) Date de dépôt: 20.06.1991
(51) Int. Cl.: C07C 33/46

(54) **PROCEDE DE PREPARATION 1,1-BIS(CHLOROPHENYL)-2,2,2 TRICHLOROETHANOL**
VERFAHREN ZUR HERSTELLUNG VON 1,1-BIS(CHLORPHENYL)-2,2,2-TRICHLORETHANOL
METHOD FOR PREPARING 1,1-BIS(CHLOROPHENYL) 2,2,2-TRICHLOROETHANOL

(30) Priorité: 22.06.1990 FR 9007849
(43) Date de publication de la demande: 10.06.1992
(73) Titulaire: ELF ATOCHEM S.A., 92800 Puteaux (FR)
(72) Inventeur: COMMANDEUR, Raymond, F-38220 Vizille (FR); CASTELLA SOLA, Jaume, E-08911 Badalona (ES); GORNY, Bernard, F-38130 Echirolles (FR); PALENCIA ADRUBAU, Jaime, E-8911 Badalona (ES)
(86) Numéro de dépôt international: FR9100495
(87) Numéro de publication internationale: WO9200264

(56) Documents cités:
- EP-A- 0 248 954
- EP-A- 0 383 054
- EP-A- 0 409 689
- GB-A- 831 421
- US-A- 2 812 362
- US-A- 2 932 672
- CHEMICAL ABSTRACTS, volume 81,1974, Columbus, Ohio, USA, TOMAR SARMAN S. et al. "2,2,2-Trichloro-1,1-bis (4-chlorphenyl)-ethanol"

## Description

La présente invention concerne un procédé de préparation de 1,1-bis(chlorophényl)-2,2,2 trichloroéthanol. Ce produit, communément appelé "dicofol", est utile comme pesticide. 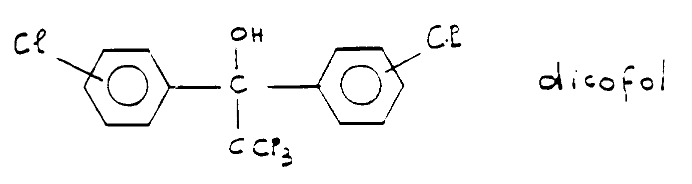
jusqu'à présent on effectuait la synthèse du dicofol à partir du DDT qui est aussi un pesticide.

Le 1,1-bis(chlorophényl) 2,2,2- trichloroéthane (DDT) cristallisé est fondu puis est transformé 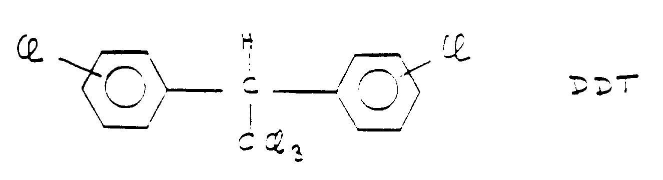
en 1,1-bis(chlorophényl) 1,2,2,2-tetrachloroéthane (chloro DDT) soit par chloration soit par une déshydrochloration suivie d'une addition de chlore puis le chloro DDT est hydrolysé en Dicofol en présence d'acide sulfurique et d'un acide sulfonique. Ce procédé est cité dans l'introduction des brevets EP 409689 et US 4705902. Les brevets US 2812280 et US 2812362 décrivent l'hydrolyse du chloro DDT en dicofol. Après l'hydrolyse on ajoute un solvant, on obtient ainsi une phase organique contenant le dicofol brut et une phase aqueuse contenant l'acide sulfurique et l'acide sulfonique. Cette phase aqueuse ne peut être réutilisée qu'une seule fois pour l'hydrolyse du chloro DDT ou dicofol. En effet, on a constaté qu'elle se chargeait en impuretés qui perturbaient l'hydrolyse. L'acide sulfonique peut être de l'acide benzènesulfonique, paratoluènesulfonique ou butylbenzènesulfonique. Cette phase acide n'est pas utilisable telle quelle, il est nécessaire de la désulfoner par de la vapeur pour faire de l'acide sulfurique résiduaire et du benzène, du toluène ou du butylbenzène. On peut ainsi récupérer du benzène, du toluène ou du butylbenzène de bonne pureté et de l'acide sulfurique résiduaire qu'on peut utiliser dans des applications ou il n'est pas nécessaire d'avoir un acide pur. Il suffit ensuite de sulfoner le benzène, toluène ou le butylbenzène pour refaire une solution d'acide sulfurique et d'acide sulfonique nécessaire à l'hydrolyse du chloro DDT.

De plus, ce procédé oblige à manipuler du DDT sous forme solide pour le mettre en solution dans le tetrachloroéthane ou dans le méthanol (exemple 2 et 3 de US 2812280). En effet, le DDT est le plus souvent livré sous forme solide en tablettes.

On a maintenant trouvé un procédé beaucoup plus simple pour préparer le dicofol qui évite la manipulation du DDT, les pertes de DDT sous forme de poussière, qui évite la préparation d'une solution d'acide sulfurique et d'acide sulfonique et donne aussi un meilleur rendement.

L'invention est un procédé de synthèse de 1,1bis(chlorophényl) 2,2,2-trichloroéthanol dans lequel :
a/ : on fait réagir du chloral avec un excès de chlorobenzène en présence d'acide sulfurique ;
b/ : on obtient une phase aqueuse contenant de l'acide sulfurique et de l'acide parachlorobenzènesulfonique et une phase organique constituée de 1,1-bis(chlorophényl) 2,2,2-trichloroéthane en solution dans le chlorobenzène ;
c/ : on transforme le 1,1-bis(chlorophényl) 2,2,2-trichloroéthane en 1,1-bis(chlorophényl) 1,2,2,2-tetrachloroéthane ;
d/ : on hydrolyse le 1,1-bis(chlorophényl) 1,2,2,2-trichloroéthane en 1,1-bis(chlorophény|) 2,2,2-trichloroéthanol en présence de tout ou partie de la phase aqueuse acide de l'étape b/ ;
e/ : on ajoute en fin d'étape d/ du chlorobenzène, on obtient ainsi une phase organique constituée de 1,1-bis(chlorophényl) 2,2,2-trichloroéthanol en solution dans le chlorobenzène et une phase aqueuse contenant de l'acide sulfurique et de l'acide parachlorobenzènesulfonique ;
f/ : les phases aqueuses acides provenant des étapes b/ et e/ sont distillées par entraînement à la vapeur pour donner (i) du chlorobenzène qu'on recycle et (ii) de l'acide sulfurique résiduaire.

La réaction de l'étape a/ est connue dans son principe, elle a été décrite dans US 2932672. "en présence d'acide sulfurique" veut dire qu'on peut avoir toute concentration y compris l'acide sous forme d'oléum. La réaction de condensation du chlorobenzène sur le chloral se fait de préférence entre -20 et -15°C. On peut ensuite chauffer vers 40 à 75°C et éventuellement rajouter du chlorobenzène jusqu'à obtenir deux phases, une organique constituée du chlorobenzène et contenant tout le 1,1bis(chlorophényl),2,2,2-trichloroéthane et une phase aqueuse contenant de l'acide sulfurique et de l'acide parachlorobenzènesulfonique. De l'eau a été formée par la condensation du chlorobenzène sur le chloral. L'acide parachlorobenzènesulfonique s'est formé par action du milieu sulfurique sur le chlorobenzène. L'homme de métier peut ajuster l'excès de chlorobenzène pour obtenir ces deux phases organique et aqueuse. La phase aqueuse peut être lavée au chlorobenzène pour être sur de bien extraire tout le 1,1bis(chlorophényl) 2,2,2-trichloroéthane. Ce chlorobenzène peut être réutilisé à l'étape a/.

La phase organique peut être lavée à l'eau ou avec de l'eau contenant du carbonate de soude.

La transformation de l'étape c/ du 1,1bis(chlorophényl) 2,2,2-trichloroéthane en 1,1,bis(chlorophényl)1,2,2,2-tetrachloroéthane peut se faire par tout moyen. C'est à dire qu'on peut déshydrochlorer puis chlorer ou directement chlorer ; le principe de ces réactions est connu en lui-même.

Par contre, la demanderesse a trouvé qu'il n'était plus nécessaire de passer par le 1,1bis(chlorophényl) 2,2,2-trichloroéthane à l'état solide.

Selon la présente invention, on peut effectuer l'étape c/ directement sur la phase organique obtenue à l'étage b/.

On peut utiliser le même procédé que celui décrit dans les exemples 2 et 3 du brevet US 2812 280 mais avec le chlorobenzène au lieu de tetrachloroéthane ou méthanol comme solvant du 1,1bis(chlorophényl) 2,2,2-trichloroéthane.

Selon une forme préférée de l'invention, on élimine de la phase organique de l'étape b/ le chlorobenzène par hydrodistillation entre 90 et 110°C, on obtient ainsi le 1,1bis(chlorophényl) 2,2,2-trichloroéthane à l'état fondu, puis on effectue une déshydrochloration à l'acide d'une solution aqueuse alcaline. On obtient une phase organique qui est constituée de 1,1bis(chlorophényl)-2,2-dichloroéthylène et une phase aqueuse alcaline.

Le 1,1bis est soumis à une chloration en masse pour produire le 1,1bis(chlorophényl) 1,2,2,2-tetrachloroéthane.

L'étape d/ du procédé de l'invention est dans son principe connue en soi, la demanderesse a trouvé qu'en utilisant en tout ou en partie la phase aqueuse acide provenant de l'étape b/ on obtenait de façon inattendue un meilleur rendement en dicofol, c'est à dire que la quantité de 1,1bis(chlorophényl),2,2,2-trichloroéthanol représente 87 à 88% en poids du produit obtenu à l'étape e/ (hors chlorobenzène), alors que si on fabrique du dicofol à partir de 1,1bis(chlorophényl) 2,2,2trichloroéthane en utilisant une phase aqueuse acide ne provenant pas de la synthèse de l'étape a/, la quantité baisse de 87/88% à 85/86%.

A cet avantage s'ajoute aussi un meilleur rendement sur le chloral.

Il était connu, après la réaction d'hydrolyse de l'étape d/ d'ajouter un solvant pour extraire le 1,1bis(chlorophényl)2,2,2-trichloroéthanol, c'est à dire de l'obtenir dans une phase organique, l'autre phase étant aqueuse et contenant les acides sulfurique et sulfonique. La demanderesse a trouvé qu'il était très simple d'utiliser le chlorobenzène. On n'utilise donc qu'un seul solvant dans tout le procédé de l'invention.

Le procédé de l'étape f/ est connu en soi. Le chlorobenzène récupéré peut être recyclé en tout point du procédé où il est utilisé, par exemple, à l'étape a/, à l'étape e/ ou pour le lavage de la phase aqueuse de l'étape b/.

On ne sortirait pas du cadre de l'invention, si on ne soumettait à l'étape f/ qu'une partie des phases aqueuses obtenues aux étapes b/ et e/ et qu'on recyclait ces phases acides aux étapes a/ et d/.

L'homme de métier ajuste la quantité de phase aqueuse acide à soumettre à l'étape f/ en fonction des performances obtenues dans le procédé de l'invention.

L'avantage de la synthèse de dicofol selon l'invention est qu'on évite une opération de cristallisation et de fusion du DDT technique. L'opération de cristallisation est difficile car le DDT passe d'abord par une phase mielleuse. Elle se fait sur une bande métallique ce qui constitue une technique insalubre et qui est source de pollution. De plus, on évite le transport d'une matière dangereuse (DDT).

Par ailleurs, ce procédé permet d'avoir un seul composé aromatique, le chlorobenzène, qui joue :
- le rôle de réactif et de solvant dans la condensation du chloral et du chlorobenzène ;
- le rôle de solvant dans la synthèse du dicofol. En effet, l'addition de chlorobenzène est utile en fin de réaction d'hydrolyse de l'étape d/ pour permettre la décantation et séparation phase organique et phase acide.

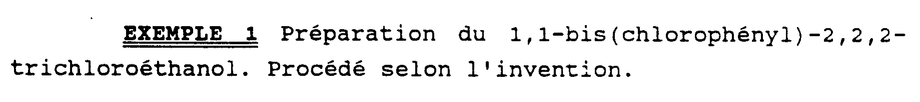

On dissout 430 kg de chloral dans 770 kg de monochlorobenzène. on ajoute 305 kg d'acide résiduaire d'une opération précédente et on refroidit à -2°C. On coule 960 kg d'oléum 22% en 14 h. La température en fin de coulage est de 20°C. On ajoute 138 kg de monochlorobenzène et la réaction est maintenue encore 4h en laissant monter la température de 20 jusqu'à 40°C.

Après l'ajout de 287 kg de monochlorobenzène, on arrête l'agitation et après 1 h de repos à 40°C, on soutire la phase acide qui se trouve à la partie inférieure, laquelle est soumise à une extraction avec 120 kg de monochlorobenzène. On obtient 1500 kg d'acide résiduaire, dont 305 kg seront chargés à l'opération suivante, 195 kg réservés à la synthèse du 1,1-bis(chlorophényl)-2,2,2-trichloroéthanol et le reste, 1000 kg réservés aussi pour sa désulfonation. La composition de cet acide résiduaire est : acide sulfurique, 59% ; acide p-chlorobenzènesulfonique, 36% ; et eau 4%.

La phase organique, constituée par le 1,1-bis(chlorophényl)-2,2,2-trichloroéthane et du monochlorobenzène, réunie avec le monochlorobenzène d'extraction de l'acide résiduaire sont lavées par 3 fois 300 kg d'eau. Au dernier lavage, on ajoute 20 kg de carbonate de soude. Le monochlorobenzène est ensuite éliminé par hydrodistillation a 110°C. On récupère 389 kg de monochlorobenzène.

Le 1,1-bis (chlorophényl)-2,2,2-trichloroéthane obtenu, à l'état liquide, est ensuite ajouté à la phase aqueuse basique d'une opération précédente de déshydrochloration et on initie la déshydrochloration avec l'ajout de 5 kg de chlorure de dimethyl benzyle lauryl ammonium tout en maintenant en agitation intense pendant 4 h à 100°C. Après 1 h de repos, on sépare la phase aqueuse, on ajoute 318 kg d'hydroxyde de soude 50% et on continue la déshydrochloration avec agitation à 100°C et 10 h. Après dilution avec 560 kg d'eau et 1 h de repos, on soutire la phase organique et on retient la phase aqueuse pour l'opération suivante. La phase organique est ensuite lavée avec 3 fois 250 kg d'une solution d'acide sulfurique 1 N et on obtient 888 kg de 1,1-bis(chlorophényl)-2,2-dichloroéthylène.

Le 1,1-bis(chlorophényl)-2,2-dichloroéthylène est soumis à une chloration en masse, avec du chlore gazeux, à 90-100°C, de lampes à vapeur de mercure et l'incorporation discontinue de 3kg d'azobisisobutyronitrile. Le chlore total introduit est de 440 kg dont 235 réagissent. Après dégazage à l'azote, on obtient 1072 kg en 1,1-bis(chlorophényl)-1,2,2,2-tetrachloroéthane.

Au 1,1-bis(chlorophényl)-1,2,2,2-tetrachloroéthane, à l'état liquide à 90°C, on ajoute 71 kg de l'eau et on chauffe à reflux. Après, on charge les 195 kg d'acide résiduaire et on continue le chauffage jusqu'à 140°C. 100 kg de chlorure obtient 1500 kg d'acide résiduaire, dont 305 kg seront chargés à l'opération suivante, 195 kg réservés à la synthèse du 1,1-bis(chlorophényl)-2,2,2-trichloroéthanol et le reste, 1000 kg réservés aussi pour sa désulfonation. La composition de cet acide résiduaire est : acide sulfurique, 59% ; acide p-chlorobenzènesulfonique, 36% ; et eau 4%.

La phase organique, constituée par le 1,1-bis(chlorophényl)-2,2,2-trichloroéthane et du monochlorobenzène, réunie avec le monochlorobenzène d'extraction de l'acide résiduaire sont lavées par 3 fois 300 kg d'eau. Au dernier lavage, on ajoute 20 kg de carbonate de soude. Le monochlorobenzène est ensuite éliminé par hydrodistillation a 110°C. On récupère 389 kg de monochlorobenzène.

Le 1,1-bis (chlorophényl)-2,2,2-trichloroéthane obtenu, à l'état liquide, est ensuite ajouté à la phase aqueuse basique d'une opération précédente de déshydrochloration et on initie la déshydrochloration avec l'ajout de 5 kg de chlorure de dimethyl benzyle lauryl ammonium tout en maintenant en agitation intense pendant 4 h à 100°C. Après 1 h de repos, on sépare la phase aqueuse, on ajoute 318 kg d'hydroxyde de soude 50% et on continue la déshydrochloration avec agitation à 100°C et 10 h. Après dilution avec 560 kg d'eau et 1 h de repos, on soutire la phase organique et on retient la phase aqueuse pour l'opération suivante. La phase organique est ensuite lavée avec 3 fois 250 kg d'une solution d'acide sulfurique 1 N et on obtient 888 kg de 1,1-bis(chlorophényl)-2,2-dichloroéthylène.

Le 1,1-bis(chlorophényl)-2,2-dichloroéthylène est soumis à une chloration en masse, avec du chlore gazeux, à 90-100°C, de lampes à vapeur de mercure et l'incorporation discontinue de 3kg d'azobisisobutyronitrile. Le chlore total introduit est de 440 kg dont 235 réagissent. Après dégazage à l'azote, on obtient 1072 kg en 1,1-bis(chlorophényl)-1,2,2,2-tetrachloroéthane.

Au 1,1-bis(chlorophényl)-1,2,2,2-tetrachloroéthane, à l'état liquide à 90°C, on ajoute 71 kg de l'eau et on chauffe à reflux. Après, on charge les 195 kg d'acide résiduaire et on continue le chauffage jusqu'à 140°C. 100 kg de chlorure d'hydrogène sont dégagés pendant la durée de la réaction d'hydrolyse de 18 h à 140-150°C. Le chlorure d'hydrogène est absorbé avec de l'eau à acide chlorhydrique. Au cours de la réaction, on ajoute 44 kg d'eau pour compenser la consommation à la réaction.

En maintenant l'agitation , on ajoute 101 kg du monochlorobenzène et 142 kg d'eau. Après 1 h de repos, on soutire la phase organique. On lave la phase acide avec 60 kg de monochlorobenzène et par soutirage, on sépare 395 kg de phase acide laquelle est réservée pour sa désulfonation.

Le monochlorobenzène de lavage est réuni avec la phase organique soutirée et l'ensemble est lavé 4 fois 240 kg d'eau. On distille le monochlorobenzène par entraînement à la vapeur d'eau, à 110-120°C. On récupère 141 kg de monochlorobenzène et 1000 kg de 1,1-bis(chlorophényl)-2,2,2-trichloroéthanol, avec un titre de 87/88%.

Les 1000 kg d'acide résiduaire réservés de la synthèse du 1,1-bis(chlorophényl)-2,2,2-trichloroéthane sont mélangés avec les 395 kg de la phase acide de la réaction d'hydrolyse à 1,1-bis(chlorophényl)-2,2,2-trichloroéthanol et avec 30 kg d'eau. La chaleur dégagée fait monter la température du mélange à 150°C , on chauffe jusqu'à 180°C et on introduit de la vapeur d'eau. Le monochlorobenzène résultant de la réaction d'hydrolyse de l'acide p-chlorobenzènesulfonique est entraîné par la vapeur et lavé avec une solution diluée d'hydroxyde de soude (3%). On récupère 205 kg de monochlorobenzène. La température finale de la réaction est de 205°C.

Tout le monochlorobenzène récupéré tout au long du procédé est directement recyclable sans besoin d'être purifié.

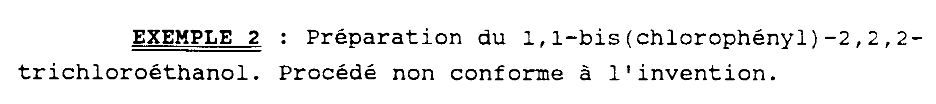

On dissout 473 kg de chloral dans 889 kg de monochlorobenzène. On coule 981 kg d'oléum 22% en 1 h 15 mn, en laissant monter la température de -2°C jusqu'à 15°C, on ajoute 181 kg de monochlorobenzène et la réaction est maintenue encore 4 h à 15°C. On ajoute 165 kg de monochlorobenzène et on chauffe à 60°C pendant 6 h.
Après 1 h de repos à 60°C, on soutire la phase acide qui se trouve à la partie inférieure , laquelle est soumise à une extraction avec 150 kg de monochlorobenzène. On obtient 1200 kg d'acide résiduaire ayant la composition suivante : acide sulfurique, 57% ; acide p-chlorobenzènesulfonique, 37% et eau 5%. Cet acide résiduaire est désulfoné à 180-205°C par introduction de la vapeur d'eau. On récupère 212 kg qui après lavage avec une solution diluée d'hydroxyde de soude (3%) sont recyclés.

La phase organique, constituée par le 1,1-bis(chlorophénil)-2,2,2-trichloroéthane et du monochlorobenzène, réunie avec le monochlorobenzène d'extraction de l'acide résiduaire sont lavés 3 fois 350 kg d'eau. Au dernier lavage, on ajoute 20 kg de carbonate de soude. Le monochlorobenzène est ensuite séparé par hydrodistillation à 110°C. On récupère 370 kg de monochlorobenzène.

Le 1,1bis-(chlorophényl)-2,2,2,-trichloroéthane fondu est ensuite solidifié, obtenant 990 kg.

Sur la phase aqueuse basique d'une opération précédente de déshydrochloration, on charge les 990 kg de 1,1bis-(chlorophényl)-2,2,2,-trichloroéthane, solide. La durée de la charge est de 2 h afin d'éviter la formation de masses solides à l'intérieur du réacteur. On ajoute 5 kg de chlorure de dimethyl benzyle lauryl ammonium tout en maintenant en agitation intense pendant 4 h à 100°C. Après 1 h de repos, on sépare la phase aqueuse et on ajoute 318 kg d'hydroxyde de soude 50% et on continue la déshydrochloration avec agitation à 100°C et 10 h. Après dilution avec 560 kg d'eau et 1 h de repos, on soutire la phase organique et on retient la phase aqueuse pour l'opération suivante. La phase organique est ensuite lavée avec 3 fois 250 kg d'une solution d'acide sulfurique 1 N et on obtient 888 kg de 1,1bis-(chlorophényl)-2,2,-trichloroéthylène.

Le 1,1bis-(chlorophényl)-2,2,-trichloroéthylène est soumis à une chloration en masse, avec du chlore gazeux, à 90-100°C, de lampes à vapeur de mercure et l'incorporation discontinue de 3 kg d'azobisisobutyronitrile. Le chlore total introduit est de 440 kg dont 235 réagissent. Après dégazage à l'azote, on obtient 1072 kg en 1,1-bis(chlorophényl)-1,2,2,2-tétrachloroéthane.

Au 1,1-bis(chlorophényl)-1,2,2,2-tétrachloroéthane à l'état liquide ) 90°C, on ajoute 71 kg d'eau et on chauffe à reflux. Après, on charge 195 kg d'un mélange d'acide sulfurique, 60,5% ; acide p-chlorobenzènesulfonique, 35% ; et eau 45%, obtenu par réaction à 100°C de l'acide sulfurique 98% et du monochlorobenzène. On continue à chauffer jusqu'à 140°C. 100 kg de chlorure d'hydrogène sont dégagés pendant la durée de la réaction d'hydrolyse de 18 h à 140-155°C. Le chlorure d'hydrogène est absorbé avec de l'eau à acide chlorhydrique. Au cours de la réaction, on ajoute 44 kg d'eau pour compenser la consommation à la réaction.

En maintenant l'agitation, on ajoute 101 kg du monochlorobenzène et 42 kg d'eau. Après 1 h de repos, on soutire la phase organique. On lave la phase acide avec 60 kg de monochlorobenzène.

Le monochlorobenzène de lavage est réuni avec la phase organique soutirée et l'ensemble est lavé 4 fois 240 kg d'eau. On distille le monochlorobenzène par entraînement à la vapeur d'eau, à 110-120°C On récupère 141 kg de monochlorobenzène et 1000 kg de 1,1-bis(chlorophényl)-2,2,2-trichloroéthanol, avec un titre de 85-86%.

## Revendications

1. Procédé de synthèse de 1,1-bis(chlorophényl)2,2,2-trichloroéthanol dans lequel :
a/ : on fait réagir du chloral avec un excès de chlorobenzène en présence d'acide sulfurique.
b/ : On obtient une phase aqueuse contenant de l'acide sulfurique et de l'acide parachlorobenzènesulfonique et une phase organique constituée de 1,1-bis(chlorobenzène)-2,2,2-trichloroéthane en solution dans le chlorobenzène.
c/ : On transforme le 1,1-bis(chlorophényl)-2,2,2-trichloroéthane en
1,1-bis(chlorophényl)-1,2,2,2-trétrachloroéthane.
d/ : On hydrolyse le
1,1-bis(chlorophényl)-2,2,2-trétrachloroéthane en présence de tout ou partie de la phase aqueuse acide de l'étape b/.
e/ : on ajoute enfin l'étape d/ du chlorobenzène. On obtient ainsi une phase organique constituée de 1,1-bis(chlorophényl)2,2,2-trichloroéthanoi en solution dans le chlorobenzène et une phase aqueuse contenant de l'acide sulfurique et de l'acide parachlorobenzènesulfonique.
f/ : la phase aqueuse acide est distillée par entraînement à la vapeur pour donner (i) du chlorobenzène, qu'on recycle et (ii) de l'acide sulfurique résiduaire.

2. Procédé selon la revendication 1 caractérisé en ce que dans l'étape c/ on élimine le chlorobenzène de la phase organique puis on effectue la déshydrochloration du 1,1bis(chlorophényl)2,2,2-trichloroéthane à l'acide d'une solution aqueuse alcaline et ensuite une chloration.

## Claims

1. Process for the synthesis of 1,1-bis(chlorophenyl)-2,2,2-trichloroethanol, in which:
a/: chloral is reacted with an excess of chlorobenzene in the presence of sulphuric acid;
b/: an aqueous phase containing sulphuric acid and para-chlorobenzenesulphonic acid and an organic phase consisting of 1,1-bis(chlorophenyl)-2,2,2-trichloroethane in solution in chlorobenzene are obtained;
c/: the 1,1-bis(chlorophenyl)-2,2,2-trichloroethane is converted to 1,1-bis(chlorophenyl)-1,2,2,2-tetrachloroethane;
d/: the 1,1-bis(chlorophenyl)-1,2,2,2-tetrachloroethane is hydrolysed in the presence of all or part of the acidic aqueous phase from step b/;
e/: at the end of step d/, chlorobenzene is added; an organic phase consisting of 1,1-bis(chlorophenyl)-2,2,2-trichlorethanol in solution in chlorobenzene and an aqueous phase containing sulphuric acid and para-chlorobenzensulphonic acid are thus obtained; and
f/: the acidic aqueous phase is steam-distilled to give (i) chlorobenzene, which is recycled, and (ii) residual sulphuric acid.

2. Process according to Claim 1, characterized in that in step c/ the chlorobenzene is removed from the organic phase and the dehydrochlorination of 1,1-bis-(chlorophenyl)-2,2,2-trichloroethane with the aid of an alkaline aqueous solution and then a chlorination are then carried out.

## Patentansprüche

1. Verfahren zur Synthese von 1,1-Bis(chlorphenyl)-2,2,2-trichlorethanol, in dem man:
a) Chloral in Gegenwart von Schwefelsäure mit einem Überschuß an Chlorbenzol reagieren läßt,
b) eine wäßrige Phase, enthaltend Schwefelsäure und p-Chlorbenzolsulfonsäure, sowie eine organische Phase, bestehend aus in Chlorbenzol gelöstem 1,1-Bis(chlorbenzol)-2,2,2-trichlorethan erhält,
c) das 1,1-Bis(chlorphenyl)-2,2,2-trichlorethan in 1,1-Bis(chlorphenyl)-1,2,2,2-tetrachlorethan umwandelt,
d) das 1,1-Bis(chlorphenyl)-1,2,2,2-tetrachlorethan in Gegenwart der kompletten wäßrigen sauren Phase aus Schritt b) oder eines Teils davon hydrolysiert,
e) am Ende des Schrittes d) das Chlorbenzol hinzufügt, so daß eine organische Phase, bestehend aus in Chlorbenzol gelösten, 1,1-Bis(chlorphenyl)-2,2,2-trichlorethanol sowie eine wäßrige Phase, enthaltend Schwefelsäure und p-Chlorbenzolsulfonsäure, gewonnen wird,
f) die wäßrige saure Phase durch Wasserdampfdestillation austreibt, um (i) Chlorbenzol, das wieder zurückgeführt wird, und (ii) die restliche Schwefelsäure zu erhalten.

2. Verfahren nach Anspruch 1, dadurch gekennzeichnet, daß man in Schritt c) das Chlorbenzol aus der organischen Phase entfernt, dann die Dehydrochlorierung des 1,1-Bis(chlorphenyl)-2,2,2-trichlorethans mit Hilfe einer wäßrigen alkalischen Lösung und anschließend eine Chlorierung durchführt.
